# EUROPÄISCHE PATENTSCHRIFT

(11) **EP 0 840 621 B1**
(45) Veröffentlichungstag und Bekanntmachung des Hinweises auf die Patenterteilung: **23.01.2002**
(21) Anmeldenummer: 96928368.8
(22) Anmeldetag: 22.07.1996
(51) Int. Cl.: A61K 47/10, A61K 47/12, A61K 9/70

(54) **HORMONPFLASTER**
HORMONE PATCH
EMPLATRE LIBERANT DES HORMONES

(30) Priorität: 22.07.1995 DE 19526864
(43) Veröffentlichungstag der Anmeldung: 13.05.1998
(73) Patentinhaber: LABTEC GESELLSCHAFT FüR TECHNOLOGISCHE FORSCHUNG UND ENTWICKLUNG MBH, 40764 Langenfeld (DE)
(72) Erfinder: CORDES, Günter, D-42799 Leichlingen (DE); SIEGMUND, Martin, D-51371 Leverkusen (DE)
(74) Vertreter: König, Beate, Dipl.-Phys. Dr.
(86) Internationale Anmeldenummer: EP9603229
(87) Internationale Veröffentlichungsnummer: WO9703698

(56) Entgegenhaltungen:
- WO-A-95/02404
- WO-A-96/08229
- WO-A-96/08255
- CHEMICAL ABSTRACTS, vol. 118, no. 24, 14.Juni 1993 Columbus, Ohio, US; abstract no. 240659, XP002022406 & PROC. PROGRAM INT. SYMP. CONTROLLED RELEASE BIOACT. MATER..18TH, 1991, Seiten 533-534, D. KATZ ET AL.: "ENHANCED SKIN PERMEATION OF PRAZOSIN BY TRANSCUTOL-OLEIC ACID MIXTURE."

## Beschreibung

Die vorliegende Erfindung betrifft ein auf der Haut anzubringendes Pflaster, auch transdermales therapeutisches System (TTS) genannt.

Hautpflaster zur transdermalen Verabreichung von Arzneistoffen sind bekannt. Ein Problem bei diesen Zubereitungen ist in der Rescrption ausreichend großer Wirkstoffmengen pro Flächen- und Zeiteinheit zu sehen, weil viele Arzneistoffe, die in einer topischen Zubereitung auf die Haut aufgetragen oder aufgeklebt werden, nicht in ausreichender Menge durch die Haut hindurchgelangen.

Aus diesem Grunde hat man sogenannte Resorptionsbeschleuniger, auch Penetrationsverbesserer, Resorptionsförderer oder Enhancer genannt, erforscht und diese in Hautpflaster eingearbeitet. Auf diese Weise war es oft erst möglich, die gewünschte pharmakologische Wirkung zu erzielen. Beispiele für Resorptionsbeschleuniger sind Propylenglykol, Polyethylenglykole niedrigeren Molekulargewichtes, Ölsäure, Isopropylmyristat, Myritol, Transcutol "Gattefossé", Eutanol "Henkel", Glycerinmonolaurat (= Imvitor 312 "Hüls"), Ricinolsäurepartialglycerid (= Softigen 701 "Hüls"), ungesättigte polyglykolisierte Glyceride (= Labrafil M1944CS "Gattefossé"), Labrafac Hydro WL1219 "Gattefossé", Estasan GT60, gesättigte polyglykolisierte Glyceride (= Labrasol "Gattefossé"), Phospholipide usw. Weitere Literatur dazu: Rieg-Falson, F. et al. 1989, Watkinson, A. C. et al. 1991 und Hadgraft, J. and Guy, R. H. (eds.): Marcel Dekker Inc. N.Y. 1989.

Aus WO-A-96/08 255 ist bereits ein Transdermales Therapeutisches System (TTS) mit einer Trägerfolie und mit einem Haftkleber auf Acrylatbasis mit einem Hormongehalt und einem Gehalt an mehreren Resorptionsbeschleunigern bekannt, wobei
- der Hormonsehalt durch einen Gehalt an Levonorgestrel gegeben ist und wobei
- es sich bei den Resorptionsbeschleunigern u. a. um eine C₈₋₂₂-Fettsäure, wie Ölsäure (Seite 6 Zeile 25), oder 2-(2-Ethoxyethoxy)-ethanol handeln kann; ein Gemisch aus Ölsäure und 2-(2-Ethoxyethoxy)-ethanol per se ist nicht genannt.

Haftkleber auf Acrylatbasis gehören zum Stand der Technik, wie beispielsweise auf Basis von DUROTAK^{R}, erhältlich durch radikalische Polymerisation von Butylacrylat, 2-Ethylhexylacrylat, Methylacrylat, Vinylacetat, Acrylsäure und/oder Hydroxyethyl-acrylat; vergl. Monomer-Liste für beispielsweise DUROTAK^{R} 280-2287.

Gegenüber diesem Stand der Technik ist Gegenstand der Erfindung ein Transdermales Therapeutisches System (TTS) mit einer Trägerfolie, mit einem Haftkleber auf Acrylatbasis mit einem Hormongehalt und einem Gehalt an Resorptionsbeschleunigern und mit einer Schutzfolie, wobei
- der Hormongehalt durch einen Gehalt an einem Estrogen und/oder Gestagen und/oder Androgen gegeben ist und wobei
- es sich bei den Resorptionsbeschleunigern um die beiden Substanzen Ölsäure und 2-(2-Ethoxyethoxy)-ethanol handelt.

Die vorliegende Erfindung betrifft also ein auf der Haut anzubringendes Pflaster, auch transdermales therapeutisches System (TTS) genannt, in dem bis zu drei Arzneistoffe enthalten sind, die aus dem TTS durch die Haut in den Körper (systemischen Kreislauf) des Menschen oder des Tieres abgegeben werden. Das Hautpflaster besteht aus einer Trägerfolie, einem Haftkleber, in dem sich die Arzneistoffe sowie weitere Substanzen, die z. B. als Resorptionsbeschleuniger dienen, befinden. Zusätzlich ist gegebenenfalls eine weitere Klebeschicht angebracht sowie eine Schutzfolie, die vor Gebrauch des Pflasters durch Abziehen entfernt wird.

Diese Erfindung betrifft also ferner die Verwendung eines Gemisches der beiden Substanzen Ölsäure und 2-(2-Ethoxyethoxy)ethanol gemeinsam in einem TTS als Enhancer für Estrogene wie Estradiol und Ethinylestradiol, Gestagene wie Norethisteronacetat, Levonorgestrel, Chlormadinonacetat und/oder Androgene wie Testosteron.

Als vorteilhaft ist außerdem noch hervorzuheben, daß es sich um zwei bekannte und als Hilfsstoffe in pharmazeutischen Zubereitungen übliche und unbedenkliche Stoffe handelt, und nicht um neue chemische Stoffe, die das Risiko von Nebenwirkungen in sich bergen würden und durch langdauernde toxikologische Untersuchungen geprüft werden müßten, bevor sie in einem Medikament zum menschlichen Gebrauch eingesetzt werden könnten.

Die Erfindung betrifft ferner ein Transdermales Therapeutisches System, das dadurch gekennzeichnet ist, daß es sich bei dem Estrogen um Estradiol oder Ethinylestradiol und bei dem Gestagen um Norethisteronacetat, Levonorgestrel, Progesteron oder Chlormadinonacetat und bei dem Androgen um Testosteron handeln kann, wobei diese Hormone auch in Form anderer Salze oder Ester oder auch der Basen eingesetzt sein können.

Das erfindungsgemäße Transdermale Therapeutische System kann dadurch gekennzeichnet sein, daß der Haftkleber auf Acrylatbasis erhalten worden ist durch radikalische Polymerisation von Eutylacrylat, 2-Ethylhexylacrylat, Methylacrylat, Vinylacetat, Acrylsäure, Hydroxyethylacrylat oder aus Mischungen einiger oder aller aufgeführten Monomere und/oder daß Vernetzer und/oder andere Hilfssubstanzen in einer Menge von weniger als 2 % hinzugefügt worden sind.

Das erfindungsgemäße Transdermale Therapeutische System kann ferner dadurch gekennzeichnet sein, daß das Gewichtsverhältnis von ölsäure zu 2-(2-Ethoxyethoxy)-ethanol 2:1 bis 1:2, vorzugsweise 1,5:1 bis 1:1,5 ist und die Menge dieses Gemisches 1 bis 10 Gew.-% beträgt, bezogen auf das TTS-Gewicht einschließlich aller Wirk- und Hilfsstoffe, jedoch ohne die Folien wie Trägerfolie und Abziehfolie.

Das erfindungsgemäße Transdermale Therapeutische System kann ferner dadurch gekennzeichnet sein, daß es
- Estradiol und Norethistercnacetat oder
- Estradiol und Levonorgestrel oder
- Ethinylestradiol und Levonorgestrel oder
- Testosteron enthält.

Im Rahmen unserer Arbeiten bei der Entwicklung von Hautpflastern mit verschiedenen Arzneistoffen wurde eine größere Anzahl Penetrationsverbesserer in die Rezeptur eingearbeitet und die Wirkstofffreisetzung untersucht. Diese wurde in der USP Paddle-Apparatur geprüft (beschrieben in der United States Pharmacopoe XXIII), und es wurde die Penetration durch "hairless mouse skin" im Modell der Franz-Zelle untersucht (Franz-Zelle : Franz, T.J., J. Invest. Dermatol. 1975 (64), p. 191-155) sowie durch menschliches Stratum Corneum, ebenfalls in der Franz-Zelle. Unter den penetrationsfördernden Substanzen befand sich Ölsäure DAB 10, 2-(2-Ethoxyethoxy)ethanol, Eutanol G (DAB 10), Labrafac, Labrafil sowie Mischungen aus Ölsäure/2-(2-Ethoxyethoxy)ethanol. Ölsäure/Labrafil, und Ölsäure/Labrafac etwa im Verhältnis 1:1.

Die Wirkstofffreigabe über die Zeit zeigte in der USP Paddle Apparatur keine großen Unterschiede.

### Bei Messung der Permeation

durch Tierhaut, besonders aber durch menschliches Stratum Corneum zeigte sich, daß Hautpflaster, die mit einer Mischung von Ölsäure und 2-(2-Ethoxyethoxy)ethanol etwa im Verhältnis 1:1 als Enhancer hergestellt wurden, erheblich höhere Permeationsraten ergaben, als dieses bei Zusatz der einzelnen Enhancer oder ohne Enhancer zu beobachten war. Überraschend war, daß die Mischung aus Ölsäure und 2-(2-Ethoxyethoxy)ethanol höhere Permeationsraten lieferte als wenn Mischungen aus Ölsäure und anderen Enhancern eingesetzt wurden. Offensichtlich zeichnet sich speziell das eine Gemisch durch besondere resorptionsbeschleunigende Eigenschaften aus.

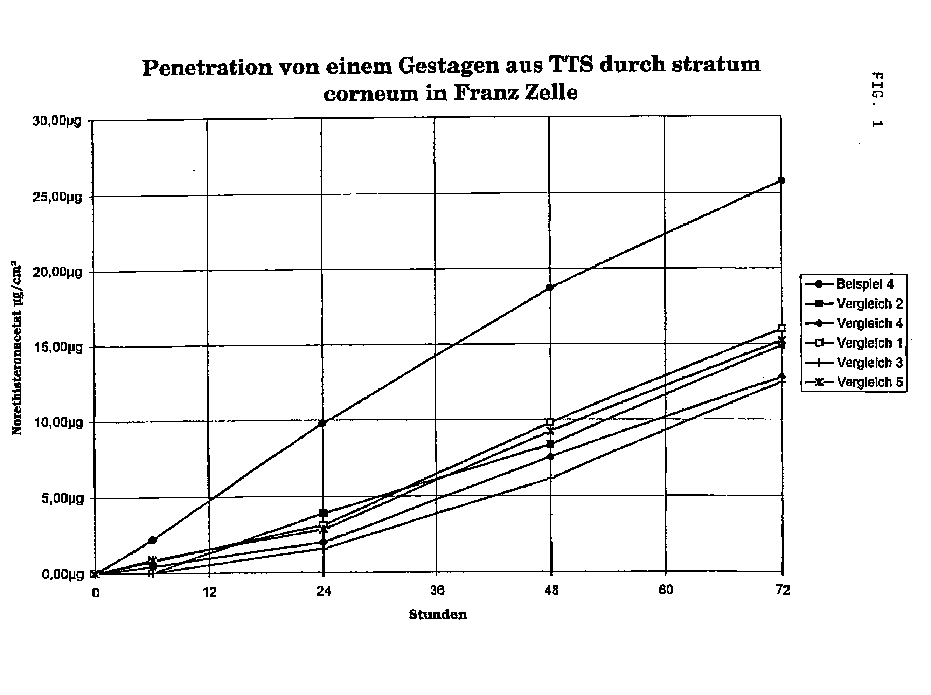

In Abb. 1 sind die Ergebnisse der Wirkstofffreisetzung und die Ergebnisse der Permeationsversuche dargestellt. Zur Analyse des Wirkstoffgehaltes im Akzeptormedium wurde eine HPLC-Kethode angewendet, wie sie dem Fachmann bekannt ist.

## Patentansprüche

1. Transdermales Therapeutisches System (TTS) mit einer Trägerfolie, mit einem Haftkleber auf Acrylatbasis mit einem Hormongehalt und einem Gehalt an Resorptionsbeschleunigern und mit einer Schutzfolie, wobei
- der Hormongehalt durch einen Gehalt an einem Estrogen und/oder Gestagen und/oder Androgen gegeben ist und wobei
- es sich bei den Resorptionsbeschleunigern um die beiden Substanzen Ölsäure und 2-(2-Ethoxyethoxy)ethanol handelt.

2. Transdermales Therapeutisches System (TTS) nach Anspruch 1, **dadurch *gekennzeichnet*, daß** es sich bei dem Estrogen um Estradiol oder Ethinylestradiol und bei dem Gestagen um Norethisteronacetat, Levonorgestrel, Progesteron oder Chlormadinonacetat und bei dem Androgen um Testosteron handelt, wobei diese Hormone auch in Form anderer Salze oder Ester oder auch der Basen eingesetzt sein können.

3. Transdermales Therapeutisches System (TTS) nach Anspruch 1 oder 2, **dadurch *gekennzeichnet*, daß** der Haftkleber auf Acrylatbasis erhalten worden ist durch radikalische Polymerisation von Butylacrylat, 2-Ethylhexylacrylat, Methylacrylat, Vinylacetat, Acrylsäure, Hydroxyethylacrylat oder aus Mischungen einiger oder aller aufgeführten Monomere und/oder daß Vernetzer und/oder andere Hilfssubstanzen in einer Menge von weniger als 2% hinzugefügt worden sind.

4. Transdermales Therapeutisches System (TTS) nach einem der Ansprüche 1 bis 3, **dadurch *gekennzeichnet*, daß** das Gewichtsverhältnis von Ölsäure zu 2-(2-Ethoxyethoxy)ethanol 2:1 bis 1:2, vorzugsweise 1/5:1 bis 1:1,5 ist und die Menge dieses Gemisches 1-10 Gew.-% beträgt, bezogen auf das TTS-Gewicht einschließlich aller Wirk- und Hilfsstoffe, jedoch ohne die Folien wie Trägerfolie und Abziehfolie.

5. Transdermales Therapeutisches System (TTS) nach einem der Ansprüche 1 bis 4, **dadurch *gekennzeichnet*, daß** Ölsäure und 2-(2-Ethoxyethoxy)ethanol etwa im Verhältnis 1:1 eingearbeitet worden sind.

6. Transdermales Therapeutisches System (TTS) nach einem der Ansprüche 1 bis 5, **dadurch *gekennzeichnet*, daß** es Estradiol und Norethisteronacetat enthält.

7. Transdermales Therapeutisches System (TTS) nach einem der Ansprüche 1 bis 5, **dadurch *gekennzeichnet*, daß** es Estradiol und Levonorgestrel enthält.

8. Transdermales Therapeutisches System (TTS) nach einem der Ansprüche 1 bis 5, **dadurch *gekennzeichnet*, daß** es Ethinylestradiol und Levonorgestrel enthält.

9. Transdermales Therapeutisches System (TTS) nach einem der Ansprüche 1 bis 5, **dadurch *gekennzeichnet*, daß** es Testosteron enthält.

## Claims

1. Transdermal therapeutic system (TTS) having a backing film, having an acrylate-based pressure-sensitive adhesive having a hormone content and a content of absorption accelerators and having a protective film, wherein:
- the hormone content is provided by a content of an oestrogen and/or gestagen and/or androgen, and wherein
- the absorption accelerators are the two substances oleic acid and 2-(2-ethoxyethoxy)-ethanol.

2. Transdermal therapeutic system (TTS) according to claim 1, **characterised in that** the oestrogen is oestradiol or ethinyl oestradlol and the gestagen is norethisterone acetate, laevonorgestrel, progesterone or chlormadinone acetate, and the androgen is testosterone, it being possible for those hormones to be used also in the form of other salts *or* esters, or also of bases.

3. Transdermal therapeutic system (TTS) according to claim 1 or claim 2, **characterised in that** the acrylate-based pressure-sensitive adhesive has been obtained by free-radical polymerisation of butyl acrylate, 2-ethylhexyl acrylate, methyl acrylate, vinyl acetate, acrylic acid, hydroxyethyl acrylate or from mixtures of some or all of the cited monomers, and/or that crosslinking agents and/or other adjuvant substances have been added in an amount of less than 2%.

4. Transdermal therapeutic system (TTS) according to any one of claims 1 to 3, **characterised in that** the ratio by weight of oleic acid to 2-(2-ethoxyethoxy)ethanol is from 2:1 to 1:2, preferably from 1.5:1 to 1:1.5, and the amount of that mixture is from 1 to 10 % by weight, based on the TTS weight including all the active ingredients and adjuvants, but without the films, such as the backing film and the removable film.

5. Transdermal therapeutic system (TTS) according to any one of claims 1 to 4, **characterised in that** oleic acid and 2-(2-ethoxyethoxy)ethanol have been incorporated in a ratio of approximately 1:1.

6. Transdermal therapeutic system (TTS) according to any one of claims 1 to 5, **characterised in that** it comprises oestradiol and norethisterone acetate.

7. Transdermal therapeutic system (TTS) according to any one of claims 1 to 5, **characterised in that** It comprises oestradiol and laevonorgestrel.

8. Transdermal therapeutic system (TTS) according to any one of claims 1 to 5, **characterised in that** it comprises ethinyl oestradiol and laevonorgestrel.

9. Transdermal therapeutic system (TTS) according to any one of claims 1 to 5, **characterised in that** it comprises testosterone.

## Revendications

1. Système thérapeutique transdermique (TTS) avec une feuille support, avec une colle de contact à base d'acrylate, ayant une teneur en hormone et une teneur en accélérateurs de résorption et avec une feuille de protection, dans lequel
- la teneur en hormone est donnée par une teneur en oestrogène et/ou gestagène et/ou androgène, et dans lequel
- quant aux accélérateurs de résorption, il s'agit des deux substances, l'acide oléique et le 2-(2-éthoxyéthoxy)éthanol.

2. Système thérapeutique transdermique (TTS) selon la revendication 1, **caractérisé en ce que**, quant à l'oestrogène, il s'agit de l'oestradiol ou de l'éthynyloestradiol et quant au gestagène, il s'agit de l'acétate de noréthistérone, du lévonorgestrel, de la progestérone ou de l'acétate de chlormadinone et quant à l'androgène,il s'agit de la testostérone, ces hormones pouvant également être mises en oeuvre sous forme d'autres sels ou esters ou également des bases.

3. Système thérapeutique transdermique (TTS) selon la revendication 1 ou 2, **caractérisé en ce que** la colle de contact à base d'acrylate a été obtenue au moyen d'une polymérisation par voie radicalaire de l'acrylate de butyle, de l'acrylate de 2-éthylhexyle, de l'acrylate de méthyle, de l'acétate de vinyle, de l'acide acrylique, de l'acrylate d'hydroxyéthyle, ou à partir de mélanges de quelques uns ou de tous les monomères énumérés et/ou **en ce que** des agents de réticulation et/ou d'autres substances auxiliaires ont été ajoutées en une quantité inférieure à 2 %.

4. Système thérapeutique transdermique (TTS) selon l'une quelconque des revendications 1 à 3, **caractérisé en ce que** le rapport pondéral de l'acide oléique au 2-(2-éthoxyéthoxy)éthanol est de 2:1 à 1:2, de préférence de 1,5:1 à 1:1,5 et la quantité de ce mélange est de 1 à 10 % en poids, par rapport au poids du TTS, incluant tous les ingrédients actifs et adjuvants, mais sans les feuilles comme, la feuille support et la feuille détachable.

5. Système thérapeutique transdermique (TTS) selon l'une quelconque des revendications 1 à 4, **caractérisé en ce que** l'acide oléique et le 2-(2-éthoxyéthoxy)éthanol ont été introduits approximativement dans le rapport 1:1.

6. Système thérapeutique transdermique (TTS) selon l'une quelconque des revendications 1 à 5, **caractérisé en ce qu'**il contient de l'oestradiol et de l'acétate de noréthistérone.

7. Système thérapeutique transdermique (TTS) selon l'une quelconque des revendications 1 à 5, **caractérisé en ce qu'**il contient de l'oestradiol et du lévonorgestrel.

8. Système thérapeutique transdermique (TTS) selon l'une quelconque des revendications 1 à 5, **caractérisé en ce qu'**il contient de l'éthinyloestradiol et du lévonorgestrel.

9. Système thérapeutique transdermique (TTS) selon l'une quelconque des revendications 1 à 5, **caractérisé en ce qu'**il contient de la testostérone.
